# EUROPEAN PATENT APPLICATION

(11) **EP 0 653 162 A1**
(43) Date of publication of application: **17.05.1995**
(21) Application number: 94916417.2
(22) Date of filing: 31.05.1994
(51) Int. Cl.: C12N 1/14, A01N 63/04

(54) **COLLETOTRICHUM AND USE THEREOF**

(30) Priority: 03.06.1993 JP 156359/93
(71) Applicant: JAPAN TOBACCO INC., Shinagawa-ku, Tokyo 140 (JP)
(72) Inventor: NISHINO, Tomoki, Japan Tobacco Inc. Applied Plant, Midori-ku, Yokohama-shi, Kanagawa 227 (JP); MIYABE, Katsuhiro Japan Tobacco Inc Applied Plant, Midori-ku, Yokohama-shi, Kanagawa 227 (JP)
(74) Representative: Reinhard, Horst, Dr.
(86) International application number: JP9400862
(87) International publication number: WO9428723

(57) **Abstract**

A strain of the genus *Colletotrichum* having a pathogenicity on poas, which is sprayed on poas such as *Poa annua* to control the same.

## Description

### FIELD OF THE INVENTION

The present invention relates to a strain belonging to the genus Colletotrichum, an agent for killing plants of the genus Poa and a method for killing plants of the genus Poa by the use of said strain, which is particularly effective for killing troublesome weed annual bluegrass (Poa annua L.) in turf.

### BACKGROUND OF THE INVENTION

Annual bluegrass, a troublesome weed in turf e.g. in a golf course, a park etc., has been controlled mainly by means of chemical herbicides by and sometimes pulling out it by hand in a golf course.

Recently, frequently applying a large amount of chemical herbicides in a golf course etc. causes an environmental pollution and organic farming without the use of any chemicals draws consumer's attention. Under the circumstances, biological pesticides are widely studied with interest because of less influence on the environment. In herbicide, plant-pathogenic organisms have been studied as the means of controlling weeds. The principal features of plant-pathogenic organisms as agents for herbicides lie in less adverse effects on crop plants and the least influence on the environment. As a conventional weed-killer intended for use in turf, mention may be made of a plant-pathogenic bacterium Xanthomonas campestris suppressing the propagation of annual bluegrass i.e. a troublesome weed of the family Gramineae (Japanese Laid-Open Patent Application No. 502438/1988). At present, Devine® (a product of Abbott Labs) for killing Stranglervine (Morrenia odorata) and Collego® (a product of Ecogen Inc.) for killing northern jointvetch (Aeschynomene virginica) are commercially available in the United States.

A wide variety of means have been attempted in the prior art as described to control annual bluegrass.

Whereas the control of the weeds by pulling out them by hand needs much labor, the control by a chemical herbicide does not need much labor, but there is not only a danger of environmental pollution etc. but also the adverse effect of the chemical on the turf, so the chemical herbicide can be used only before (or at the early stage of) the germination of annual bluegrass, and it is not effective to grown annual bluegrass.

In contrast, the control by the plant-pathogenic bacterium Xanthomonas campestris does not need much labor and is effective to grown annual bluegrass, too. However, since Xanthomonas campestris (NRRL-B-18078) is a bacterium, it is necessary for the target weed annual bluegrass to be artificially cut for its application. Furthermore, a relatively higher temperature of 28 to 30°C is required for the considerable inhibition of the growth of annual bluegrass.

Hence, there has been demand for a means of controlling the troublesome weed annual bluegrass, in which there is no danger of environmental pollution etc.; grown annual bluegrass can also be controlled; and its application is easy without requiring any tiresome procedures.

### SUMMARY OF THE INVENTION

As a result of their extensive study on the means of killing the troublesome weed Poa annua of the family Gramineae in turf, the present inventors found a fungus which is pathogenic to Poa annua, but is not pathogenic to useful plants including rice (Oryza sativa).

That is, the present invention encompasses:
1. A strain belonging to the genus Colletotrichum which is pathogenic to plants of the genus Poa but is not pathogenic to useful plants.
2. A strain belonging to the species Colletotrichum sp. P-191 which is pathogenic to plants of the genus Poa but is not pathogenic to useful plants.
3.Colletotrichum sp. P-191 strain (FERM BP-4264) which is pathogenic to plants of the genus Poa but is not pathogenic to useful plants.
4. An agent for killing plants of the genus Poa comprising an active ingredient a strain belonging to the genus Colletotrichum which is pathogenic to plants of the genus Poa but is not pathogenic to useful plants.
5. An agent for killing plants of the genus Poa according to item 4 above wherein the strain belonging to the genus Colletotrichum is a strain belonging to the species Colletotrichum sp. P-191.
6. An agent for killing plants of the genus Poa according to item 5 above wherein the strain belonging to the species Colletotrichum sp. P-191 is Colletotrichum sp. P-191 strain (FERM BP-4264).
7. A method for killing plants of the genus Poa wherein a Poa-killing agent comprising as an active ingredient a strain belonging to the genus Colletotrichum which is pathogenic to plants of the genus Poa but is not pathogenic to useful plants is applied to plants of the genus Poa.
8. A method for killing plants of the genus Poa according to item 7 above wherein the strain belonging to the genus Colletotrichum is a strain belonging to the species Colletotrichum sp. P-191.
9. A method for killing plants of the genus Poa according to item 8 above wherein the strain belonging to the genus Colletotrichum sp. P-191 is Colletotrichum sp. P-191 strain (FERM BP-4264).

### DETAILED DESCRIPTION OF THE INVENTION

The useful plants referred to in the present invention are those useful as turf or edible crops, and examples are Gramineae plants (excluding plants of the genus Poa), e.g. plants of the genus Oryza such as rice etc., plants of the genus Triticum such as wheat etc., plants of the genus Zea such as corn etc., plants of the genus Agrostis such as bentgrass etc. , in addition to Leguminous plants such as kidney beans etc., Solanaceae plants such as eggplant, tomato etc.

Hereinafter, the microorganism of the present invention is described.

P-191 strain is a fungus which is pathogenic to annual bluegrass but is not pathogenic to useful plants including rice etc., and it was isolated in pure culture from the infected tissues of the diseased annual bluegrass collected from various locations in Japan.

P-191 strain, when cultured in a medium, shows the following characteristics.

It grows well on a potato dextrose agar medium (PDA; pH 5.6) and Lima bean agar medium (LBA; pH 5.6). If mycelial plug of 5 mm diameter precultured on a PDA plate is inoculated onto a fresh PDA plate and cultured at 25°C, it will grow about 90 mm diameter about 2 weeks after inoculation. The mycelia on the medium is dark green to dark brown in the center and pale pink therearound with colorless hyphae at the top. Conidia are one-celled, and crescent-shaped. Annual bluegrass plants, when infected with P-191 strain, exhibit brown spots on stems and leaves.

From the microscopic observations that its conidia are one-celled ① and hyaline ②, and light-orange, slimy masses of spores accumulated on the acervuli ③, and that acervuli with setae were found in the diseased Poa annua, P-191 strain was identified to the genus Colletotrichum as a result of the comparison of these characteristics with known fungi with reference to Makoto Hiura: "Shokubutsu Byogenkinrui Kaisetsu" (Guide to Plant-Pathogenic fungi), the first edition published by Yokendo on December 10, 1964. Because the species of the present strain was difficult to identify, Colletotrichum sp. P-191 was assigned as the species name.

P-191 strain was deposited under No. FERM BP-4264 on April 16, 1993 with the National Institute of Bioscience and Human-Technology, Agency of Industrial Science and Technology in Tsukuba City, Ibaraki Prefecture, Japan.

The culture of P-191 strain can be effected without any special procedures in an analogous way to that of a known microorganism belonging to the genus Colletotrichum.

Any synthetic medium and natural medium can be used insofar as it contains suitable amounts of an assimilable carbon source, nitrogen source, inorganic source and required growth-promoting substances. Examples are potato dextrose medium, potato dextrose agar medium, Lima bean medium, V8 juice agar medium, potato sucrose agar medium, Czapek-Dox medium, corn meal agar medium, oat meal agar medium, etc., among which potato dextrose agar medium and Lima bean agar medium are preferably used. The carbon source used includes glucose, sucrose, etc., and the nitrogen source used includes nitrates (NaNO₃,KNO₃) etc. The medium may additionally contain yeast extract, amino acids, and vitamins as necessary. The culture temperature is 16-33°C, preferably 18-25°C, and the pH is 5-8, preferably 5.5-6.5.

The spores are produced on the medium usually in 6-10 days incubation, though depending on the culture conditions. The spores can be easily recovered without any special means, for example by scratching the surface of the medium with sterilized water poured thereonto. The number of the produced spores varies depending on the culture conditions, typically about 10⁶ spores in a petri dish of 90 mm diameter.

Hereinafter, the weed-killing agent and method of the present invention are described.

The weed-killing agent of the present invention can be applied in a wide variety of forms, but usually in the form of an aqueous spore suspension. The microorganism used herein is not limited to the above-described P-191 strain and it may be any strain belonging to the genus Colletotrichum and being pathogenic to plants of the genus Poa but not pathogenic to useful plants.

The concentration of spores in the weed-killing agent is not particularly limited insofar as plants of the genus Poa can be effectively killed, but usually in the range of 10⁴-10⁶ spores/ml, preferably 10⁵-10⁶ spores/ml. Besides fresh spores just after incubated, stored spores may be reconstituted in an aqueous medium.

To prepare such an aqueous suspension, auxiliary agents such as a surfactant, a spreading agent, etc., may also be added to the weed-killing agent. The surfactant may be ionic or non-ionic, and mention may be made of Tween 80, Triton X-100 and Silwet L-77. As the spreading agent, mention may be made of agar, sodium alginate and xanthan gum.

The subject of the weed-killing agent and method of the present invention is a plant of the genus Poa, and particularly annual bluegrass (Poa annua L.) can be effectively killed or controlled.

The method of the present invention is effected by spreading the weed-killing agent onto plants of the genus Poa.

Spreading of the weed-killing agent can be effected without requiring any special procedures in an analogous way to that of a conventional weed-killing microorganisms. Whereas the procedure of cutting the target weeds before application is required in the case of the above-mentioned weed-killing agent comprising Xanthomonas campestris as the active ingredient, this is not required in the present invention because the microorganism of the present invention is fungus.

The time and season for spreading the present weed-killing agent are not particularly limited, and it can be used regardless of the growth conditions of the target weeds. That is, since the microorganism of the present invention selectively kills plants of the genus Poa, the time for applying the present weed-killing agent is not limited as opposed to the conventional chemical herbicide which is required to be applied before (or at the early stage of) the germination of plants of the genus Poa; and since the microorganism of the present invention can grow well at a considerably low temperature, the season for applying the present weed-killing agent is not limited to the hot season during which the above-mentioned weed-killing agent comprising Xanthomonas campestris as the active ingredient should be used.

The amount of the agent spread is not particularly limited as far as plants of the genus Poa can be effectively killed, but preferably in the range of 3.5× 10⁸ to 3.5× 10¹⁰ spores, more preferably3.5× 10⁹ to 3.5× 10¹⁰ spores, per 10 ares.

### EXAMPLES

The present invention is described in more detail with reference to the following examples, which however are not intended to limit the present invention.

### (1) Isolation and selection of the genus Colletotrichum

Diseased annual bluegrass plants in parks or golf courses were collected. Several small sections approximately 5 mm square were cut from the margin of the infec lesion to contain both diseased and healthy-looking tissue. These were surface-sterilized for 1-3 min. in a 1:1 mixture of sodium hypochloride (0.5 % effective chlorine) and 0.1 % Tween 80.

The tissue sections were washed once with 0.1 % Tween 80, then twice with sterilized water, and placed on potato dextrose agar medium supplemented with antibiotics (hereinafter referred to as APDA). They were incubated at 25°C and mycelial tip growing from the tissues were transferred onto fresh PDA plates.

Furthermore, the diseased annual bluegrass plants collected from turf were stored as herbarium specimens, and the lesions were cut off and placed under moist condition at 25°C to yield mycelia and spores.

Spores obtained by either method, were isolated under a stereoscope and pure culture was obtained on PDA plates.

The strains thus isolated are shown in Table 1.

**Table 1**

| strain | pathogenicity |
|---|---|
| P-191 | +++ |
| KZ-a | ++ |
| KZ-b | ++ |
| KZ-c | ++ |
| U-a | ++ |
| K-c | ++ |
| MS-b | ++ |
| H-20a | +++ |
| KH-a | ++ |
| HS-a | + |
| M-12a | + |
| K-d | + |

Each strain was examined for its pathogenicity to annual bluegrass as well as to rice, wheat, kidney bean, eggplant, tomato, and bentgrass. Among the tested strains, excellent control on annual bluegrass was obtained from P-191 strain snd this fungus was not pathogenic to rice, wheat, kidney bean, eggplant, tomato, and bentgrass.

The selected P-191 strain was inoculated onto potato dextrose agar medium and cultured at 25 °C.

### (2) Identification of P-191 strain

P-191 strain was cultured on PDA medium. For identification, the morphological features of the spores and sporulation structure were inspected, and the symptoms in the host plants infected with P-191 strain were also inspected.

The spores or mycelia of P-191 strain were plated onto PDA medium, and incubated at 25 °C under continuous light for about 1 week to yield a large amount of spores (about 10⁶ spores per petri dish.)

### (3) Preparation of annual bluegrass-killing agents

The P-191 strain spores selected in (1) above were washed with 0.1 % Tween 80 and re-suspended in 0.1 % Tween 80 to 10⁴, 10⁵ and 10⁶ spores/ml, respectively, then each spore suspension was used as the annual bluegrass-killing agent.

### (4) A method of killing weeds by the use of the annual bluegrass-killing agent

Seeds of annual bluegrass were sowed in a plastic pot of 75 mm × 75 mm. About 2 weeks thereafter, each pot was sprayed with 2 ml of the Poa annua-killing agent prepared in (3) above.

### Test Examples

### (1) Effect on annual bluegrass

Seeds of Poa annua were sowed into a 75 mm× 75 mm plastic pot and grown for approximately 2 weeks.

Each spore suspension prepared in Example (3) was used as the annual bluegrass-killing agent.

In the same manner described in Example (4), each pot was sprayed with 2 ml of the annual bluegrass-killing agent prepared in Example (3), and the sprayed plants were placed in a dew chamber at 25 °C.

12, 24, and 48 hours after dew treatment, the annual bluegrass plants were moved to a greenhouse and monitored for disease developed.

The results are shown in Table 2.

**Table 2**

| Dew Periods | spore concentrations | | |
|---|---|---|---|
| | 10⁴ spores/ml | 10⁵ spores/ml | 10⁶ spores/ml |
| 12 hours | 5 % | 15 % | 40 % |
| 24 hours | 10 % | 90 % | 100 % |
| 48 hours | 30 % | 100 % | 100 % |
| 100 % indicates complete kill. | | | |

As can be seen from Table 2, the annual bluegrass-killing agent containing P-191 strain controlled annual bluegrass, and excellent control was obtained at a concentration of higher than 10⁵ spores/ml.

### (2) Effect on crop plants products and turfgrass

The crop plants examined were rice, wheat, kidney bean, eggplants and tomato, and the turfgrass examined was bentgrass.

A spore suspension of P-191 strain was prepared at a concentration of 10⁶ spores/ml and examined in the same manner as in item (1) above except that dew period was 48 hours.

The results are shown in Table 3.

**Table 3**

| test plant | pathogenicity |
|---|---|
| annual bluegrass | +++ |
| rice | - |
| wheat | - |
| bentgrass | - |
| tomato | - |
| kidney bean | - |
| eggplant | - |
| "+" indicates pathogenicity. "-" indicates no pathogenicity. | |

As can be seen from Table 3, P-191 strain is not pathogenic to rice, wheat, kidney bean, eggplant, tomato and bentgrass.

## Claims

1. A strain belonging to the genus Colletotrichum which is pathogenic to plants of the genus Poa but is not pathogenic to useful plants.

2. A strain belonging to the species Colletotrichum sp. P-191 which is pathogenic to plants of the genus Poa but is not pathogenic to useful plants.

3. Colletotrichum sp. P-191 strain (FERM BP-4264) which is pathogenic to plants of the genus Poa but is not pathogenic to useful plants.

4. An agent for killing plants of the genus Poa comprising an active ingredient a strain belonging to the genus Colletotrichum which is pathogenic to plants of the genus Poa but is not pathogenic to useful plants.

5. An agent for killing plants of the genus Poa according to claim 4 wherein the strain belonging to the genus Colletotrichum is a strain belonging to the species Colletotrichum sp. P-191.

6. An agent for killing plants of the genus Poa according to claim 5 wherein the strain belonging to the species Colletotrichum sp. P-191 is Colletotrichum sp. P-191 strain (FERM BP-4264).

7. A method for killing plants of the genus Poa wherein a Poa-killing agent comprising as an active ingredient a strain belonging to the genus Colletotrichum which is pathogenic to plants of the genus Poa but is not pathogenic to useful plants is applied to plants of thegenus Poa.

8. A method for killing plants of the genus Poa according to claim 7 wherein the strain belonging to the genus Colletotrichum is a strain belonging to the species Colletotrichum sp. P-191.

9. A method for killing plants of the genus Poa according to claim 8 wherein the strain belonging to the genus Colletotrichum sp. P-191 is Colletotrichum sp. P-191 strain (FERM BP-4264).
